# EUROPÄISCHE PATENTANMELDUNG

(11) **EP 4 197 575 A1**
(43) Veröffentlichungstag der Anmeldung: **21.06.2023**
(21) Anmeldenummer: 22205383.7
(22) Anmeldetag: 03.11.2022
(51) Int. Cl.: A61M 5/31, A61M 5/20

(54) **SMARTES INJEKTIONSGERÄT MIT ERGONOMISCHER ANZEIGE**

(30) Priorität: 14.12.2021 CH 0707092021
(71) Anmelder: Ypsomed AG, 3401 Burgdorf (CH)
(72) Erfinder: Jordi, Christoph, 5000 Aarau (CH); Jost, Reto, 3506 Grosshöchstetten (CH)
(74) Vertreter: Meier Obertüfer, Jürg

(57) **Zusammenfassung**

Die Erfindung betrifft ein smartes Injektionsgerät, zum Beispiel einen Autoinjektor, mit elektronischen Elementen und einer ergonomischen Anzeige mit verschiedenen Anzeigebereichen. Die Anzeige ist dabei so unterteilt, dass eine nutzende Person vor, während und nach eines Injektionsvorgangs jeweils den relevanten Teil der Anzeige gut einsehen kann.

## Beschreibung

### TECHNISCHES GEBIET

Die vorliegende Erfindung betrifft das Gebiet von Injektionsgeräten zur Verabreichung von fluiden, insbesondere flüssigen, Medikamentenformulierungen. Speziell betrifft die Erfindung das Gebiet der smarten Injektionsgeräte.

### HINTERGRUND DER ERFINDUNG

Aus der EP 2892590 B1 sind ausgeklügelte elektronisch gesteuerte Injektionsgeräte bekannt. Die beschriebenen Geräte sind wiederverwendbar, lediglich das Injektionsmodul 132 muss ausgetauscht werden. Die Zeichnungen der Schrift zeigen ein kastenförmiges Gehäuse 102, welches von einer nutzenden Person beim Verabreichen einer Dosis Medikament mit der Hand gehalten wird, siehe dazu zum Beispiel Figur 61a. Auf der breiten Frontfläche der Geräte ist der Bildschirm 246 angeordnet, welcher Informationen und Anweisungen ausgeben. So kann der Bildschirm eine benutzende Person schrittweise durch die Vorbereitung und die Durchführung des Injektionsprozesses führen, siehe dazu die Absätze [0412] bis [450]. Weiter umfassen die beschriebenen Injektionsgeräte an ihrem hinteren Ende einen visuellen Indikator 128 oder 828, in Form einer Leuchtdiode, welche verschiedene Zustände der Injektionsgeräte vor und während des Injektionsvorgang anzeigen kann (durch leuchten, blinken etc.), es wird wieder auf die Absätze [0412] bis [0450] der EP 2892590 B1 verwiesen, welche hiermit durch Verweis vollständig in die vorliegende Schrift aufgenommen wird. Die beschriebenen Geräte haben viele Vorteile und können anwendenden Person eine erhebliche Hilfe bieten, indem sie die Person durch den ganzen Verabreichungsprozess hindurch führen und auch die Verabreichung selber automatisiert durchführbar ist. Wie jedoch aus den zahlreichen Figuren des Patents eindeutig zu entnehmen ist, handelt es sich bei den beschrieben Injektionsgeräten um komplexe Geräte, ja eigentlich Handheld-Computer. Der Bildschirm zeigt Anweisungen in Textform an, aufgrund von Textgrösse und Spiegelungen auf der Oberfläche der Geräte kann der jeweils angezeigte Text für die benutzende Person mit einer Beeinträchtigung schwierig abzulesen sein. Aufgrund der vorhandenen zahlreichen Tasten (270, 272,274, 112, 116) stellt die Bedienung für ältere Menschen, welche nicht mit elektronischen Geräten gross geworden sind, eine richtige Herausforderung dar. Aufgrund der wenig ergonomischen Kastenform ist das Halten der beschriebenen Injektionsgeräte während der Injektion entweder einfach unangenehmen oder je nach Disposition einer nutzenden Person fast unmöglich.

Es besteht deshalb ein ungesättigtes Bedürfnis nach elektronischen Injektionsgeräten, welche die beschriebenen Vorteile behalten und jedoch die Nachteile beseitigen können.

Der Begriff "Produkt", "Medikamentenformulierung", "Medikament" oder "medizinische Substanz" umfasst im vorliegenden Zusammenhang jede fliessfähige medizinische Formulierung, welche geeignet ist zur kontrollierten Verabreichung mittels einer Kanüle oder Hohlnadel in subkutanes oder intramuskuläres Gewebe, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP 1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

Der Begriff "distal" bezeichnet eine zum vorderen, einstechseitigen Ende der Verabreichungsvorrichtung beziehungsweise zur Spitze der Injektionsnadel hin gerichtete Seite oder Richtung. Demgegenüber bezeichnet die Angabe "proximal" eine zum hinteren, dem einstechseitigen Ende gegenüberliegenden Ende der Verabreichungsvorrichtung hin gerichtete Seite oder Richtung.

Unter den Begriffen "Injektionssystem", "Injektionsgerät", "Injektionsvorrichtung" oder "Injektor" wird in der vorliegenden Beschreibung eine Vorrichtung verstanden, bei der die Injektionsnadel nach erfolgter Abgabe einer kontrollierten Menge der medizinischen Substanz aus dem Gewebe entfernt wird. Somit verbleibt bei einem Injektionssystem oder bei einem Injektor im Unterschied zu einem Infusionssystem die Injektionsnadel nicht über einen längeren Zeitraum von mehreren Stunden im Gewebe.

### DARSTELLUNG DER ERFINDUNG

Es ist eine Aufgabe der Erfindung, moderne Injektionsgeräte mit Elektronik bereitzustellen, welche Nutzer und Nutzerinnen in einfacher und ergonomischer Art und Weise durch einen Injektionsprozess leiten können.

Die Aufgabe wird gelöst durch Injektionsgeräte gemäss dem unabhängigen Anspruch 1. Vorteilhafte Weiterbildungen der Erfindungen sind den abhängigen Ansprüchen, der Beschreibung und den Figuren zu entnehmen.

In einem Aspekt betrifft die Erfindung ein Injektionsgerät. Wie viele konventionelle Injektionsgeräte, wie der NovoPen 6 von Novo Nordisk oder der Ypsomate von Ypsomed, hat auch ein erfindungsgemässes Injektionsgerät eine stiftähnliche Form. Das erfindungsgemässe Injektionsgerät umfasst ein Gehäuse, welches die äussere Form abbildet. Das Gehäuse hat ein vorderes (distal) und ein hinteres Ende (proximal). Dazwischen ist eine Mantelfläche angeordnet. Im hinteren Bereich der Mantelfläche (zum hinteren Ende hin) ist eine sogenannte Grifffläche auf der Mantelfläche angeordnet. Die Grifffläche kann dabei ein Teil oder Bereich der Mantelfläche sein oder als separates Teil auf der Mantelfläche fixiert sein. Die Grifffläche dient dazu, der Person, welche das Injektionsgerät benutzt, das Halten des Injektionsgerätes zu erleichtern, zum Beispiel mit weichen Rippen, welche quer zur Achse des Injektionsgerätes um die Mantelfläche herum verlaufen. Die Grifffläche erstreckt sich in axiale Richtung in etwa entsprechend der Breite einer menschlichen Hand, in etwa 8-12 Zentimeter, so dass die nutzende Person die Grifffläche mit kleinem, Ring-, Mittel- und Zeigefinger gut greifen kann. Alternativ kann sich die Grifffläche auch über einen kürzeren Bereich erstrecken, zum Beispiel so, dass nur Zeige- und Mittelfinger im Bereich der Grifffläche greifen. Im Bereich der Grifffläche ist die Mantelfläche bevorzugt ergonomisch geformt, besonders bevorzugt nicht zylinderförmig sondern mit entlang der Achse variablem Querschnitt senkrecht zur Achse, also beispielsweise konkav oder konvex. Die Querschnittsfläche selbst ist bevorzugt annähernd kreisrund, kann aber alternativ auch ein Viereck mit abgerundeten Ecken sein.

Das erfindungsgemässe Injektionsgerät umfasst ein Reservoir für das Medikament, welches typischerweise ganz im Gehäuse angeordnet ist. Das Reservoir kann zum Beispiel eine vorbefüllte Glasspritze oder eine Karpule sein. Andere Formen sind möglich. Zur Verabreichung des Medikaments ist am vorderen Ende des Injektionsgeräts eine Injektionskanüle angeordnet, welche in Fluidverbindung mit dem Reservoir ist. Ist das Reservoir eine vorbefüllte Glasspritze, so ist die Kanüle schon fest an der Spritze angeordnet. Ist das Reservoir eine Karpule, kann die Kanüle typischerweise am vorderen Ende des Gehäuses angebracht werden, oder in gewissen Fällen auch direkt an der Karpule. Die Kanüle durchsticht dann mit einem hinteren Ende eine Dichtungsmembran (Septum) der Karpule, um die Flüssigkeitsverbindung herzustellen. Am vorderen Ende des Injektionsgeräts kann eine bewegliche Nadelschutzhülse angebracht sein. Das Injektionsgerät umfasst weiter eine Antriebsvorrichtung, welche ebenfalls im Gehäuse angeordnet ist. Typischerweise umfasst der Antrieb eine Kolbenstange, welche den Stopfen oder Kolben der Spritze oder der Karpule in distale Richtung verschieben kann, um Medikament durch die Kanüle hindurch zu verabreichen. Die Kolbenstange kann dabei manuell, durch eine Feder oder durch einen Motor (mit oder ohne Getriebe) angetrieben und bewegt werden. Dem Fachmann sind die Varianten zum Antrieb hinlänglich bekannt. Weiter umfasst die Injektionsvorrichtung elektronische Komponenten. Insbesondere eine elektronische Steuereinheit, Anzeigeelemente sowie Sensorik und optional eine Kommunikationsvorrichtung. Bei der Steuereinheit kann es sich um einen Mikrokontroller oder eine kleine CPU handeln und. Mit der Steuereinheit verbunden sind allfällige Sensoren sowie allfällige Kommunikationsvorrichtungen. Mit der Steuereinheit weiter verbunden sind die Anzeigeelemente, welche in der Erfindung eine zentrale Rolle spielen. Die Anzeigeelemente fungieren als eine Schnittstelle zwischen nutzender Person und dem Injektionsgerät.

Erfindungsgemäss ist mindestens ein Anzeigeelement auf der Mantelfläche und mindestens ein Anzeigeelement am hinteren Ende angeordnet. Bei den Anzeigeelementen sind für die nutzende Person leuchtende, durchscheinende oder transparente Piktogramme (Icons) oder Piktogramm-Umrissflächen auf der Mantelfläche des Gehäuses sichtbar, und leuchtende Leuchtdioden (LEDs) mit einfachen, unspezifischen Leuchtflächenformen am hinteren Ende des Injektionsgerätes. Es können zum Beispiel Lichtleiter verwendet werden, um dem Licht der LEDs eine adäquate geometrische Form zu verpassen, wie zum Beispiel eine Ring- oder Kreuzform. Die leuchtenden Piktogramme können durch eine entsprechende Gestaltung der Gehäusewandungen und/oder durch aufgeklebte Folien mit transparenten und/oder opaken Bereichen gestaltet werden, welche mit LEDs angestrahlt werden. Alternative können auch farbige LEDs oder mehrfarbige LEDs eingesetzt werden. Die Wandungsbereiche können auch farbig durchscheinend gestaltet sein. So kann zum Beispiel ein blau-weisses Bluetooth-Logo in der Wandung angeordnet werden, welches dann von innerhalb des Gehäuses angestrahlt wird, so dass die nutzende Person das Logo (Piktogramm) gut erkennen kann. Am erfindungsgemässen Injektionsgerät sind mehrere Anzeigeelemente angeordnet und sie bilden, wie erwähnt, die Schnittstelle zur nutzenden Person, das heisst, sie werden zur Kommunikation unterschiedlicher Zustände des Injektionsgerät zur nutzenden Person verwendet. Deshalb sind die Anzeigeelemente an zwei unterschiedlichen Bereichen des Gehäuses angeordnet, so dass eine optimale Lesbarkeit der richtigen Anzeigeelemente vor und während der Injektion für die nutzende Person gewährleistet werden kann. Vor einer Injektion kann es je nach Art der Injektion notwendig sein, verschiedene Arbeitsschritte mit dem Injektionsgerät durchzuführen. Wenn das Injektionsgerät wegen dem im Injektionsgerät vorhandenen Medikament im Kühlschrank gelagert werden muss, so kann es zum Beispiel notwendig sein, dass das Injektionsgerät (speziell das im Injektionsgerät vorhandene Medikament) vor der Injektion auf Raumtemperatur gebracht werden muss. Dazu wird das Injektionsgerät zum Beispiel 20 Minuten vor der eigentlichen Injektion aus dem Kühlschrank genommen und auf eine Arbeitsfläche gelegt, wo das Injektionsgerät aufwärmen kann. Während dieser Phase kann erfindungsgemäss ein Anzeigeelement zum Beispiel in Form eines Piktogramms auf der Mantelfläche blinken. Nach Abschluss der Aufwärmphase, welche zeit- oder temperaturgesteuert sein kann, kann das Piktogramm dann zum Beispiel grün leuchten. Ein weiterer Vorbereitungsschritt kann das Aufbauen einer Kommunikationsverbindung zwischen dem Injektionsgerät und einem Mobilgerät wie einem Smart Phone sein. So kann zum Beispiel ein Bluetooth-Anzeigeelement, ebenfalls auf der Mantelfläche angeordnet, vor dem erfolgreichen Verbinden mit zum Beispiel einem Smart Phone blau blinken und nach erfolgreicher Verbindungsetablierung blau leuchten, so lange die Verbindung aktiv ist. Auch kann weiter ein Leuchtelement zur Anzeige bei Fehlern auf der Mantelfläche angeordnet sein, welche zum Beispiel Problemen mit dem Antrieb Orange blinkt. Die Anzeigeelemente auf der Mantelfläche weisen jeweils ein Piktogramm auf, welches auf einfache Art und sprachunabhängig den Sinn der Anzeige transportiert. Während einer Injektion umgreift die nutzende Person das Injektionsgerät im Bereich der Grifffläche und kann typischerweise die Anzeigeelement an der Mantelfläche zumindest teilweise nicht mehr sehen. Um der nutzenden Person des Injektionsgeräts die Nutzung möglichst verständlich zu gestalten ist, wie erwähnt, am hinteren Ende des Injektionsgeräts (proximales Ende), welches für die nutzende Person auch während einer Injektion gut sichtbar ist, mindestens ein weiteres Anzeigeelement angeordnet, welches seinerseits mindestens eine Leuchtdiode mit einer generischen, beispielsweise halbkugelähnlichen, domförmigen, oder klötzchenförmigen, Form umfasst. Die Leuchtdiode kann zum Beispiel eine mehrfarbige Leuchtdiode sein (RGB LED). Über das mindestens eine Anzeigeelement am hinteren Ende des Injektionsgeräts kann das Anzeigeelement während des Injektionsvorgangs durch Blinken oder Leuchten monochrom oder mit verschiedenen Farben den aktuellen Status des Vorgangs signalisieren. Zum Beispiel kann das Anzeigeelement während der Ausschüttung und optional während einer darauffolgenden Haltezeit blinken und anschliessend konstant leuchten. Alternativ kann das Element während der Ausschüttung orange leuchten und nach Ablauf der Haltezeit grün leuchten um dem Anwender zu signalisieren, dass das Injektionsgerät jetzt von der Injektionsstelle entfernt werden kann. Kontinuierliche Farb- und/oder Helligkeitsänderungen während der Ausschüttung sind auch denkbar, sogar mit einem maximalen Gradienten beim Übergang zur Haltezeit, damit der Anwender davon abgehalten wird, das Injektionsgerät vorzeitig, vor Ablauf der Haltezeit zu bewegen.

Das mindestens eine Anzeigeelement am hinteren Ende des Injektionsgeräts kann in einem Aspekt mehrteilig ausgestaltet sein. So kann eine für das Anzeigeelement genutzte LED im Inneren des Injektionsgeräts an einer Elektronikanordnung angeordnet sein. Ein Lichtleiter kann sodann genutzt werden, um von der LED emittiertes Licht zum Gehäuse im Bereich des Anzeigeelements zu leiten. Die für von aussen sichtbare Form des Anzeigeelements kann zum Beispiel kreuzförmig sein. So kann der Lichtleiter zu dieser Kreuzform geführt sein, so dass von aussen ein leuchtendes Kreuz zu sehen ist. In einem Beispiel kann die Kreuzform als transparenter oder opaker Bereich des Gehäuses ausgeführt sein. Alternativ kann die sichtbare Form des Anzeigeelements rund oder oval sein. Das Anzeigeelement kann so am hinteren Ende des Injektionsgerät angeordnet sein, dass Licht nach proximal sowie quer zur Achse des Injektionsgeräts abgestrahlt wird, was insbesondere dann vorteilhaft ist, wenn das erfindungsgemässe Injektionsgerät einerseits für Injektionen in den Oberschenkel und andererseits auch für Injektionen in das Abdomen verwendet werden kann. Weitere Alternativen sind möglich.

In einem Aspekt der Erfindung sind mehrere Anzeigeelemente im Bereich der Grifffläche, wobei die Elemente auf die Längsachse bezogen in einer Ausgestaltung auf gleicher axialer Höhe und verteilt entlang des Umfangs der Mantelfläche angeordnet sind. In einer alternativen Ausgestaltung sind die Anzeigeelemente axial übereinander auf der Mantelfläche angeordnet. In einer weiteren Ausgestaltung kann die Anordnung der Anzeigeelemente eine Mischform der zwei vorher beschriebenen Anordnungen sein.

In einem Aspekt der Erfindung werden die verschiedenen Anzeigeelemente zeitlich gestaffelt aktiviert und deaktiviert. In einer alternativen Ausgestaltung können auch mehrere Anzeigeelemente gleichzeitig aktiviert und/oder deaktiviert werden. Die zeitliche Staffelung der Aktivierungen und Deaktivierung kann dabei vorteilhaft der Anleitung der nutzenden Person dienen. Alternativ oder ergänzend kann die Staffelung auch der Information der nutzenden Person oder anderer Personen dienen.

In einem Aspekt der Erfindung können eines oder mehrere der Anzeigeelemente vor, während und/oder nach einem Injektionsvorgang auf unterschiedliche Weise aktiviert und deaktiviert werden. Zum Beispiel über unterschiedliche Blinkmuster, unterschiedliche Leuchtdauer, unterschiedliche Helligkeit oder unterschiedliche Farben.

Die Aktivierung/Deaktivierung von Leuchtdioden kann in verschiedenen Aspekten der Erfindung auf verschieden Weise erfolgen, wie schon beschrieben. Nebst Leuchten und Blinken, ist auch ein Pulsieren und/oder ein "Anschwellen" der Leuchtstärke resp. ein Fading der Leuchtstärke möglich. Auch ist es möglich, dass sich die Farbtemperatur schrittweise oder kontinuierlich ändert. Weiter ist es möglich, dass sich die Farbe schrittweise oder kontinuierlich ändert. Die Erfindung setzt hier bewusst keine Einschränkung, sondern leitet den Fachmann oder die Fachfrau zu einem dem Einsatz angemessenen Einsatz der Leuchtmittel von einem diskreten zurückhaltenden Einsatz bis zu einem extensiven Einsatz der Leuchtmittel ähnlich einer Weihnachtsbeleuchtung. Der Einsatz soll der Anwendung des Injektionsgeräts angepasst sein.

In einem Aspekt der Erfindung umfasst das erfindungsgemässe Injektionsgerät mindestens einen Sensor, dessen Signal von der Steuereinheit verarbeitet werden können. Vorteilhaft umfasst das erfindungsgemässe Injektionsgerät mehrere Sensoren. Dabei kann es sich akustische Sensoren handeln, Geräusche von innerhalb des Injektionsgeräts detektieren, zum Beispiel Klickgeräusche, wie sie beim Auslösen eines Injektionsgeräts oder nach Abschluss einer Injektion auftreten können. Alternativ oder ergänzend können auch ein oder mehrere Temperatursensoren vorhanden sein. So kann mit einem Temperatursensor die Medikamententemperatur überwacht werden. Zum Beispiel während der Vorbereitung für eine Injektion, wenn ein im Kühlschrank gelagertes Medikament vor Injektion auf Raumtemperatur aufgewärmt wird. So kann die Steuereinheit nach Erreichen der gewünschten Temperatur ein entsprechendes Anzeigeelement auf der Mantelfläche aktivieren, welches der nutzenden Person sodann signalisiert, dass die Temperatur des Medikamentes nun eine Injektion zulässt. In einer weiteren vorteilhaften Ausgestaltung ist auf der Mantelfläche mindestens ein berührungssensitiver Sensor angeordnet, über welchen die Steuereinheit erkennen kann, wenn das Injektionsgerät in der Hand gehalten wird. Weiter können auch ein oder mehrere Neigungs- und/oder Beschleunigungssensoren vorhanden sein, deren Signale von der Steuereinheit ausgewertet werden.

In einem Aspekt der Erfindung handelt es sich beim erfindungsgemässen Injektionsgerät um einen Autoinjektoren, wie er ähnlich als Ypsomate der Herstellerin Ypsomed oder als Molly der Herstellerin SHL Medical bekannt ist. Der Autoinjektor kann für das einmalige Verabreichen einer einzelnen Dosis ausgelegt sein. Alternativ kann der Autoinjektor als teilweise wiederverwendbares Injektionsgerät ausgelegt sein. Ein solches Gerät umfasst eine Spritzeneinheit zum einmaligen Gebrauch, mit einer vorgefüllten Fertigspritze, einem Spritzenhalter und einer Nadelschutzhülse wie in EP 22186390.5 beschrieben, sowie eine mehrfach verwendbare Antriebseinheit mit der Grifffläche. Der Querschnitt dieser Antriebseinheit kann, bedingt durch den Kopplungsmechanismus zwischen Spritzen- und Antriebseinheit, deutlich von einer Kreisform abweichen, also oval oder gar viereckig gestaltet sein. Denkbar ist ein rechteckiger Querschnitt (mit bevorzugt verrundeten Kanten) mit einem Kantenlängenverhältnis grösser als zwei.

In einem Aspekt der Erfindung handelt es sich beim erfindungsgemässen Injektionsgerät um einen sogenannten Patch Injector ("Pflaster-Injektor"), welcher für die Verabreichung einer einzelnen Dosis auf der Haut der nutzenden Person angebracht werden kann.

In einem Aspekt der Erfindung handelt es sich beim erfindungsgemässen Injektionsgerät um einen Injektionspen, wie er ähnlich als Unopen, Servopen oder Ypsopen der Herstellerin Ypsomed bekannt ist. Der Injektionspen kann für das Verbreichen von mehreren Dosen von Medikament in variabler oder fester Grösse ausgelegt sein. Weiter kann der Injektionspen dafür ausgelegt, dass er nach dem Entleeren eines Reservoirs entsorgt wird oder alternativ das Reservoir getauscht werden kann.

In einem Aspekt der Erfindung handelt es sich beim erfindungsgemässen Injektionsgerät um ein stiftförmiges Injektionsgerät in Form eines Autoinjektors oder Injektionspens. Das Gehäuse umfasst in diesem Aspekt mehrere, vorteilhaft zwei, Schalenteile, welche entweder das Gehäuse bilden oder an oder mit weiteren Teilen des Gehäuses kombiniert werden können. Diese Schalenteile dienen dazu, das Aussendesign des Injektionsgeräts zu variieren. Beispiele dazu sind zum Beispiel in der US2018/0126083 A1 dargelegt, welche hiermit durch Verweis vollständig in das vorliegende Dokument übernommen wird. Das Gehäuse kann in einem Beispiel ein inneres Gehäuse oder eine Trägerstruktur umfassen, in welchem die funktionalen Teile des Injektionsgeräts untergebracht sind. Das innere Gehäuse umfasst Durchbrüche für die Anzeigeelemente, insbesondere lichtdurchlässige Durchbrüche. Die Schalenteile können sodann auf dem inneren Gehäuse angebracht werden. Die Schalenteile umfassen die für die nutzende Person sichtbaren Elemente der Anzeigeelemente, wie zum Beispiel Piktogramme. Dabei sind diese Elemente so an den Schalenteilen angeordnet, dass wenn die Schalenteile am inneren Gehäuse angebracht sind, Teile der Anzeigeelemente, welche an den Schalenteilen angeordnet sind, zu den Durchbrüchen am inneren Gehäuse passend angeordnet sind. Die Schalenteile können einen Bereich oder auch die ganze Grifffläche umfassen. Die Kombination aus innerem Gehäuse und Schalenteilen wie beschrieben, ermöglicht es, grundsätzlich gleiche Injektionsgeräte über eine geometrische Anpassung der Schalenteile an unterschiedliche Anwendungen (auch unterschiedliche Medikamente) oder Designvorlieben anzupassen. Ein weiterer Vorteil der Schalenteile ist, dass das Injektionsgerät auch bezüglich der Anzeigeelemente angepasst werden können. Auch wenn die Anzeigeelemente an geometrisch identischen Orten angeordnet sind, können die Symbole/Piktogramme an den Schalenteilen variiert werden. Die Aktivierung der einzelnen Anzeigeelemente ist dann eine Frage der Programmierung der Firmware der Steuereinheit des Injektionsgeräts und kann ohne Änderung der eigentlichen Elektronik(-Hardware) angepasst werden.

In einem Aspekt der Erfindung kann das erfindungsgemässe Injektionsgerät weiter ein Kommunikationsmodul umfassen, welches die drahtlose Kommunikation zwischen dem Injektionsgerät und weiteren Geräten, wie Computern oder Smart Phones ermöglicht. Die Kommunikation kann Zustandsdaten des Injektionsgeräts umfassen. Dies kann laufend oder nur zu bestimmten Zeitpunkten erfolgen. Weiter kann über eine Kommunikation von einem Smart Phone an das Injektionsgerät zum Beispiel das Injektionsgerät für den Gebrauch entsperrt werden. Das Kommunikationsmodul wird von der Steuereinheit gesteuert. Beim Kommunikationsmodul kann es sich bei bevorzugten Ausführung der Erfindung um eine Bluetooth-Einheit handeln. Alternativ kann es sich beim Kommunikationsmodul auch um WLAN- oder NFC-Einheiten handeln. Weiter können auch mehrere Kommunikationsmodule in einem erfindungsgemässen Injektionsgerät angeordnet sein.

In einem weiteren Aspekt der Erfindung ist das erfindungsgemässe Injektionsgerät modular aufgebaut. Insbesondere Autoinjektoren werden typischerweise beim Hersteller des Geräts nicht vollständig zusammengebaut. Stattdessen übernimmt die Endmontage eine Pharmafirma oder entsprechend spezialisierte Firma, welche den Autoinjektor mit dem Medikamentenbehältnis (zum Beispiel eine vorbefüllte Spritze) und allenfalls weiteren Elementen verheiratet. Gerade bei erfindungsgemässen Geräten, welche zusätzlich zur klassischen mechanischen Verabreichungsanordnung noch elektronische und allenfalls elektrische Komponenten umfassen, lohnt es sich, die richtige Aufteilung in möglichst wenige vormontierte Baugruppen (oder Module) aufzuteilen, welche dann in der Endmontage auf einfachste Art und Weise mit dem Medikamentenbehältnis verheiratet werden können. So können zum Beispiel Elektronik und Antriebselemente, wie ein Motor, zu einer Gruppe zusammengefasst werden, allenfalls mit einem der Gehäuseteile. Idealerweise sind dann die erfindungsgemässen Anzeigeelemente, welche ja in Beziehung mit der Elektronik stehen an diesem Gehäuseteil angeordnet.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden.
- Fig. 1: zeigt den erfindungsgemässen Autoinjektor 1
- Fig. 2: zeigt den modularen Aufbau des Autoinjektors 1 aus zwei vormontierten Baugruppen
- Fig. 3: zeigt eine alternative Aufteilung der Baugruppen für den Autoinjektor 1
- Fig. 4: zeigt die alternative Aufteilung der Baugruppen mit alternativem Board 30a"
- Fig. 5: mögliches System aus Autoinjektor 1 und einem Smart Phone 2

### FIGURENBESCHREIBUNG

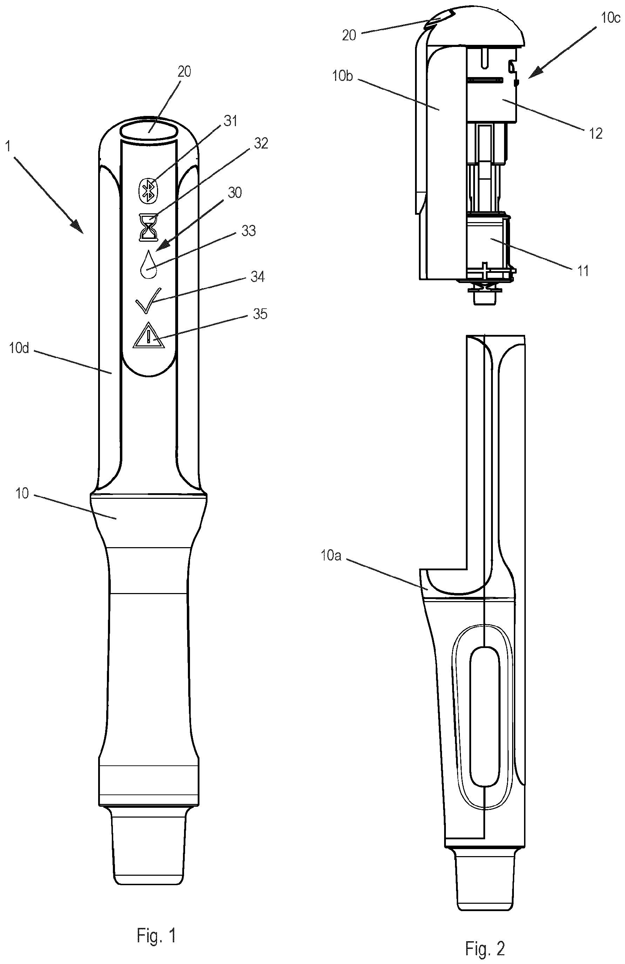

Figur 1 zeigt ein erfindungsgemässes Injektionsgerät, in der beispielhaften Form eines Autoinjektors 1. Der Autoinjektor 1 umfasst alle typischen Elemente eines Autoinjektors, wie zum Beispiel in der WO2016/205961 A1 offenbart, welche hiermit durch Verweis vollständig in das vorliegende Dokument übernommen wird. Zusätzlich umfasst der erfindungsgemässe Autoinjektor 1 Elektronik mit Steuerungseinheit, Anzeigeelementen, Kommunikationsmöglichkeiten und Sensorik. Der Antrieb kann über einen Federantrieb oder einen Motor geregelt sein.

In der Ansicht aus Figur 1, welche den Autoinjektor 1 von aussen zeigt, umfasst der Autoinjektor 1 ein Gehäuse 10, welches vorteilhaft aus mehreren fest zusammengefügten Teilen besteht. Das Gehäuse weist im proximalen Bereich den Griffbereich 10d mit der beschriebenen Grifffläche auf. Die Mantelfläche des Gehäuses ist dabei im Beispiel in mehrere sich axial erstreckende Bereiche gegliedert, wovon der Griffbereich 10d mit der Grifffläche einer ist. Wie der Name sagt, wird der Griffbereich 10d während einer Injektion typischerweise von der nutzenden Person mit einer Hand gehalten.

Im axialen Bereich des Griffbereichs 10d ist auch die erste Anzeige 30 angeordnet. In der Anzeige 30 sind die folgenden Teilanzeigen, nämlich die Bluetooth-Anzeige 31, die Wartesymbolanzeige 32, die Verabreichungsanzeige 33, die Anzeige für eine vollendete Injektion 34 sowie die Fehler-Anzeige 35, axial übereinander angeordnet. Die einzelnen Teilanzeigen in Anzeige 30 zeichnen sich durch eine piktogrammartige Gestaltung aus, welche sprachunabhängig gut gelesen werden kann. Jede der Teilanzeigen kann durch mindestens eine LED zum Leuchten gebracht werden, wobei das Leuchten von der Steuereinheit (nicht gezeigt) aus steuerbar ist.

Am hinteren Ende des Autoinjektors 1 ist eine weitere, von Anzeige 30 separierte Anzeige angeordnet, nämlich das Anzeigeelement 20. Auch das Anzeigeelement 20 kann durch mindestens eine LED (nicht gezeigt) zum Leuchten gebracht werden. Im Unterschied zu den Teilanzeigen aus Anzeige 30 strahlt jedoch das Anzeigeelement 20 nicht primär quer zur Achse des Autoinjektors 1, sondern in axiale Richtung nach proximal. Das Anzeigeelement 20 ist auch dann noch sichtbar, wenn der Autoinjektor 1 von Hand gehalten wird, während die Teilanzeigen von Anzeige 30 durch die Hand verdeckt werden können.

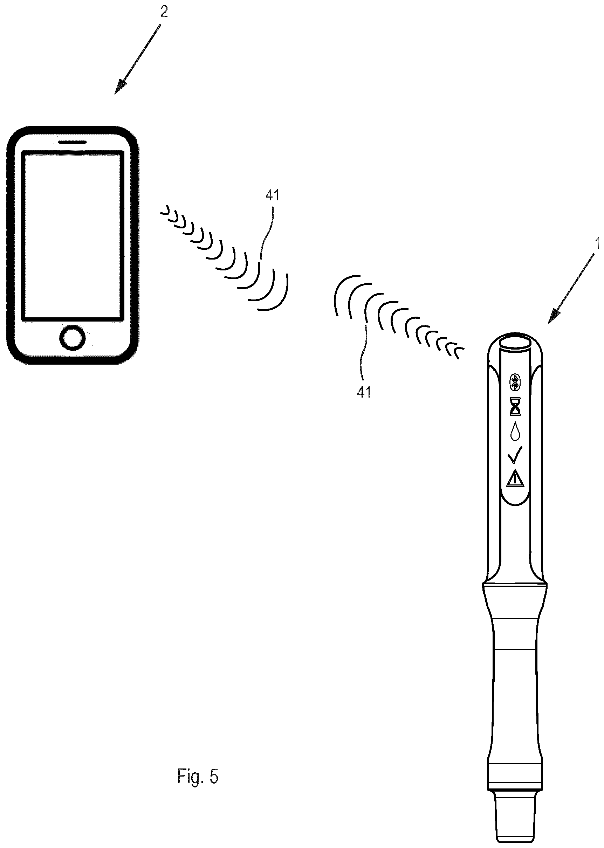

Figur 5 zeigt ein mögliches erfindungsgemässes System aus einem Smart Phone 2 und dem Autoinjektor 1, wobei der Autoinjektor 1 in dieser Ausgestaltung über ein Bluetooth-Modul verfügt, welches zur Kommunikation mit dem Smart Phone 2 über die Bluetooth-Verbindung 41 genutzt werden kann.

Die Nutzung des Autoinjektors 1 wird folgend anhand eines Beispiels erläutert. In einem ersten Schritt vor einer Injektion entnimmt die nutzende Person den Autoinjektor der Verpackung (nicht gezeigt), dabei wird der Autoinjektor 1 vorteilhaft aktiviert (sprich, die Steuereinheit wird eingeschalten) (nicht gezeigt). Im nächsten Schritt verbindet die nutzende Person den Autoinjektor 1 mit dem Smart Phone 2. Nach der Aktivierung des Autoinjektors 1 beginnt das Bluetooth-Anzeigeelement 31 zu blinken (blau) und signalisiert damit Verbindungsbereitschaft. Parallel dazu öffnet die nutzende Person die passende Therapie-App, welche zum Autoinjektor 1 passt (muss vorgängig installiert sein). Die App erkennt sodann, dass Autoinjektor 1 verbindungsbereit ist und fordert die nutzende Person auf, die Verbindung zu bestätigen. Nach dem Erhalt der Bestätigung bauen Autoinjektor und Smart Phone (und die Therapie-App) eine verschlüsselte Verbindung auf. Wenn das erfolgreich geschehen ist, wechselt das Anzeigeelement 31 von einem Blinken auf Leuchten. Im Beispiel des gezeigten Autoinjektors 1, muss das Medikament und damit der Autoinjektor im Kühlschrank gelagert werden. Vor einer Injektion sollte in diesem Beispiel das Medikament erst auf etwa Raumtemperatur gebracht werden. Um der nutzenden Person anzuzeigen, ob sie noch warten muss oder die Injektion starten kann, sind die Wartesymbol-Teilanzeige 32 sowie die Verabreichungszeige 33 vorgesehen. Solange noch mit der Injektion zugewartet werden sollte, blinkt die Wartesymbol-Teilanzeige rot, sobald die Wartezeit vorbei ist, leuchtet das Wartesymbol grün. Mit dem Abschluss der Wartezeit wird auch die Verabreichungsanzeige 33 aktiviert, welche nun grün blinkt. Das ist für die nutzende Person das Zeichen, dass sie den Autoinjektor 1 ergreifen, den allfällig vorhandenen Nadelschutz entfernen und mit der Injektion beginnen kann. Dazu setzt die nutzende Person den Autoinjektor 1 mit dem vorderen Ende (distales Ende) auf der passenden Hautstelle auf. Da die Anzeige 30 nun zumindest teilweise verdeckt ist, wird mit der Verabreichungsanzeige 33 auch die Anzeige 20 aktiviert, welche auch in diesem Zustand immer noch gut abzulesen ist. Anzeige 20 blinkt dabei in kurzen Abständen gelb auf. Die nutzende Person drückt nun den Autoinjektor gegen die Haut, wodurch die eigentliche Injektion aktiviert wird und Medikament verabreicht. Nun blinkt die Anzeige 20 grün. Nach dem Abschluss der Verabreichung hört die Anzeige 20 auf grün zu blinken, sondern leuchtet nun durchgehend grün, um der nutzenden Person zu signalisieren, dass sie den Autoinjektor 1 abnehmen kann. Nach dem Abnehmen von der Haut hört die Anzeige 20 auf zu leuchten und erlöscht. Dafür leuchtet nun die Anzeige für den Abschluss der Injektion 34 grün, was auch wieder sichtbar, weil die nutzende Person den Autoinjektor 1 unterdessen zum Beispiel auf eine Arbeitsebene abgelegt hat. Wenn jetzt während dem ganzen Prozedere ein technisches Problem am Autoinjektor 1 oder ein Fehler bei der Bedingung passiert wäre, würde zum Zeitpunkt des Fehlers die Fehleranzeige 35 und vorteilhaft während einer Injektion auch die Anzeige 20 rot zu blinken beginnen, um der nutzenden Person anzuzeigen, dass etwas nicht funktioniert hat.

Die Therapie-App kann den ganzen Ablauf begleiten oder gar Schritt-für-Schritt anleiten. Dazu werden laufend Daten zwischen dem Autoinjektor 1 und dem Smart Phone 2 ausgetauscht. So weiss die App zum Beispiel immer, in welchem Zustand der Autoinjektor 1 ist. Und kann allenfalls auch detailliertere Fehlermeldungen entgegennehmen. Auch kann die App Verabreichungsdaten weiter an einen entfernten Server geben.

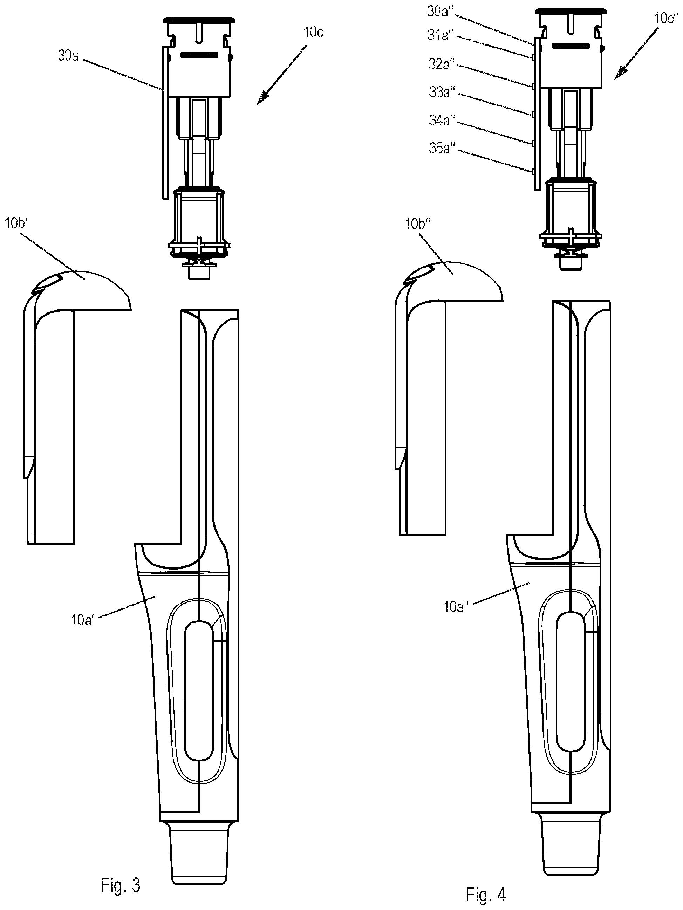

Wie schon in der allgemeinen Beschreibung dargelegt, ist das erfindungsgemässe Injektionsgerät in einem oder mehreren Aspekten der Erfindung modular aufgebaut. Dies weil die Herstellerin des Injektionsgeräts das Injektionsgerät in verschiedenen Fällen nicht vollständig montiert, bevor es zum Beispiel an eine Pharmafirma ausgeliefert wird. In solchen Fällen übernimmt zum Beispiel die Pharmafirma die Endmontage. Um eine möglichst einfache und effiziente Endmontage zu ermöglichen, wird das erfindungsgemässe Injektionsgerät bei der Geräteherstellerin in Modulen oder Baugruppen vormontiert. Die Aufteilung, welche Elemente des Injektionsgeräts in welcher Baugruppe untergebracht werden, spielt dabei eine erhebliche Rolle für die Endmontage. Die Figuren 2 und 3 zeigen zwei mögliche Aufteilungen für den Autoinjektor 1. In Figur 2 ist der Autoinjektor 1 symbolisch in die (Gehäuse-) Module 10a und 10b aufgeteilt, für die Endmontage muss sodann (zumindest) noch der Medikamentenbehälter, zum Beispiel eine vorbefüllte Spritze, in das vordere Modul 10a eingeschoben werden und zum Schluss das Modul 10b auf das Modul auf gesetzt werden. In diesem Beispiel umfasst das Gehäuse Modul 10b auch das Elektronik- und Antriebsmodul 10c mit (einem Teil) des Antriebs 11, insbesondere einer Antriebsfeder oder einem Motor, sowie die Elektronik 12 mit insbesondere der Steuerungseinheit und den Anzeigen (nicht gezeigt). In einer alternativen Ausgestaltung wie in Figur 3 gezeigt, sind das hintere Gehäusemodul 10b' und das Elektronik- und Antriebsmodul 10c' getrennte Module. In Figur 3 ist nun auf ein Board 30a für die Anzeige 30 im Bereich der Grifffläche zu sehen. Die LEDs (nicht gezeigt) für die Anzeigeelemente 31 bis 35 sind dabei im Board 30a integriert. In einer weiteren alternativen Ausgestaltung, welche in Figur 4 gezeigt wird, ist Modulaufteilung gleich wie in Figur 3, mit den Modulen 10a", 10b" sowie 10c". Anders als bei Figur 3 sind die LEDs 31a" bis 35a" für die korrespondierenden Anzeigeelemente 31 bis 35 bei dieser Ausgestaltung auf das Board 30a aufgesetzt (zum Beispiel aufgelötet).

### BEZUGSZEICHENLISTE

- 1: Autoinjektor
- 2: Smart Phone
- 10: Gehäuse
- 10a: vorderes Gehäusemodul
- 10a': vorderes Gehäusemodul
- 10a": vorderes Gehäusemodul
- 10b: hinteres Gehäusemodul
- 10c: Elektronik- und Antriebsmodul
- 10d: Griffbereich
- 11: Antrieb (Teile des Antriebs)
- 12: Elektronik
- 20: Anzeigeelement (am hinteren Ende)
- 30: Anzeige im Bereich der Grifffläche
- 30a: Board für Anzeige
- 30a": alternative Ausführung des Boards für die Anzeige
- 31: Bluetooth-Anzeige
- 31a": LED
- 32: Wartesymbol-Anzeige
- 32a": LED
- 33: Verabreichungsanzeige
- 33a": LED
- 34: Anzeige für Abschluss der Injektion
- 34a": LED
- 35: Fehler-Anzeige
- 35a": LED
- 41: Bluetooth-Verbindung

## Patentansprüche

1. Ein Injektionsgerät (1) zur Verabreichung von fluiden Medikamentenformulierungen, umfassend
a. ein vorzugsweise in etwa stiftförmiges Gehäuse (10) mit einer Mantelfläche, welche sich zwischen einem vorderen und einem hinteren Ende erstreckt, wobei das Gehäuse (10) eine Achse definiert,
b. ein Medikamentenreservoir für die Medikamentenformulierung, welches zumindest teilweise im Gehäuse (10, 10a) angeordnet ist,
c. eine Injektionskanüle, welche lösbar oder unlösbar am Injektionsgerät (1) oder dem Medikamentenreservoir befestigbar oder befestigt ist, durch welche Medikamentenformulierung aus dem Medikamentenreservoir verabreichbar ist und welche zumindest während der Verabreichung mit einem Ende über das vordere Ende des Gehäuses hinausragt,
d. eine Antriebsvorrichtung (10c), mit welcher Medikamentenformulierung aus dem Medikamentenreservoir durch die Injektionskanüle verabreichbar ist,
e. eine Grifffläche (10d) in einem hinteren Bereich der Mantelfläche, wobei der hintere Bereich an das hintere Ende des Gehäuses anschliesst und wobei die Grifffläche (10d) die Mantelfläche zumindest teilweise entlang des Umfangs der Mantelfläche umläuft,
f. elektronische Komponenten (12), welche zumindest teilweise innerhalb des Gehäuses (10) angeordnet sind, wobei die elektronischen Komponenten (12) mehrere Anzeigeelemente (31, 32, 33, 34) umfassen, welche zumindest sichtbares Licht abgeben können, wobei die Anzeigeelemente durch das Gehäuse (10) hindurch sichtbar, oder in der Mantelfläche und/oder dem hinteren Ende des Gehäuses angeordnet sind,
g. ein Speicher für elektrische Energie zum Versorgen der elektronischen Komponenten und optional der Antriebsvorrichtung,
h. wobei die elektronischen Komponenten (12) weiter eine elektronische Steuereinheit umfassen, welche die Anzeigeelemente steuern und welche Signale von Sensoren in und am Injektionsgerät empfangen kann, und
i. wobei die elektronischen Komponenten (12) weiter eine mit der elektronischen Steuereinheit verbundene Kommunikationseinheit umfassen, über welche drahtlos Daten und/oder Information von der Steuereinheit an andere Geräte kommunizierbar oder von anderen Geräten empfangbar sind,
**dadurch gekennzeichnet, dass**
j. es sich bei den Anzeigeelementen um Elemente mit Piktogrammen, um piktogrammähnliche Elemente oder um einfache Leuchtelemente aus Leuchtdiode und optional Lichtleiter handelt, und dass
k. mindestens ein Anzeigeelement (20) am hinteren Ende des Gehäuses angeordnet ist und Licht zumindest teilweise in axiale Richtung nach hinten vom Gehäuse abstrahlen kann, sowie
l. mindestens ein Anzeigeelement (31, 32, 33, 34) im Bereich der Grifffläche angeordnet ist, wobei das mindestens eine Anzeigeelement ein Piktogramm (31, 32, 33, 34) oder mindestens ein piktogrammähnliches Element umfasst und Licht in etwa radiale Richtung von der Mantelfläche abstrahlen kann.

2. Injektionsgerät nach dem vorhergehenden Anspruch, wobei mehrere Anzeigeelemente (31, 32, 33, 34) im Bereich der Grifffläche angeordnet sind und wobei mindestens eines der mehreren Anzeigeelemente ein Piktogramm (31, 32, 33, 34) oder piktogrammähnliches Element umfasst.

3. Injektionsgerät (1) nach dem vorhergehenden Anspruch, wobei die Anzeigeelemente (31, 32, 33, 34) im Bereich der Grifffläche entweder axial übereinander oder entlang des Umfangs nebeneinander angeordnet sind.

4. Injektionsgerät (1) nach dem vorhergehenden Anspruch, wobei die übereinander oder nebeneinanderliegenden Anzeigeelemente (31, 32, 33, 34) zeitlich gestaffelt aktiviert werden.

5. Injektionsgerät (1) nach dem vorhergehenden Anspruch, wobei die zeitliche Staffelung der Aktivierung der Vorbereitung der Verabreichung der fluiden Medikamentenformulierung dient.

6. Injektionsgerät (1) nach einem der vorhergehenden Ansprüche, welches am hinteren Endes des Gehäuses (810) ein oder zwei Anzeigeelemente (20) umfasst, wobei diese jeweils mindestens eine Leuchtdiode und einen Lichtleiter umfassen, wobei der Lichtleiter durch das hintere Ende des Gehäuses hindurchgeführt ist, so dass Licht, welches von der Leuchtdiode emittiert wird, über den jeweiligen Lichtleiter auf eine Aussenseite des Gehäuses geleitet wird.

7. Injektionsgerät (1) nach dem vorhergehenden Anspruch, wobei am hinteren Ende des Gehäuses (10) genau ein Anzeigeelement (20) mit einer Leuchtdiode angeordnet ist, wobei die Leuchtdiode vor, während und nach der Verabreichung auf jeweils andere Art aktiviert werden kann, und wobei das Licht, welches von der Leuchtdiode emittiert wird und vom Anzeigeelement an die Umgebung abgestrahlt wird, hauptsächlich in proximale Richtung abgestrahlt wird.

8. Injektionsgerät (1) nach dem vorhergehenden Anspruch, wobei die Leuchtdiode des am hinteren Ende des Gehäuses (10) angeordneten Anzeigeelements (20) eine mehrfarbige Leuchtdiode ist.

9. Injektionsgerät (1) nach einem der beiden vorhergehenden Ansprüche, wobei die Leuchtdiode vor, während und nach der Verabreichung in jeweils anderen Farben strahlt und/oder blinkt.

10. Injektionsgerät (1) nach Anspruch 7, wobei die Leuchtdiode, wenn das Injektionsgerät (1) bereit für die Verabreichung ist, blinkt, wobei die Leuchtdiode während der Verabreichung konstant leuchtet, und wobei die Leuchtiode nach Abschluss der Verabreichung löscht.

11. Injektionsgerät (1) nach Anspruch 7, wobei das Licht, welches von der Leuchtdiode emittiert wird und vom Anzeigeelement (20) an die Umgebung abgestrahlt wird, teilweise auch zur Seite also quer zur Achse des Injektionsgeräts abgestrahlt wird.

12. Injektionsgerät (1) nach einem der vorhergehenden Ansprüche, wobei das Injektionsgerät (1) mindestens einen Sensor umfasst, mit welchem mindestens eine Zustandsgrösse des Injektionsgeräts (1) erfassbar ist, welche mit der elektronischen Steuerungseinheit (12) verarbeitbar ist.

13. Injektionsgerät (1) nach einem der vorhergehenden Ansprüche, wobei es sich beim Injektionsgerät (1) um einen Autoinjektoren, bevorzugt um einen Einwegautoinjektor, handelt.

14. Injektionsgerät (1) nach einem der Ansprüche 1 bis 10, wobei es sich beim Injektionsgerät (1) um einen Injektionspen handelt, mit welchem mehrere Einzeldosen der Medikamentenformulierung verbreichbar sind, wobei die einzelnen Dosen eine feste oder variable Grösse aufweisen können.

15. Injektionsgerät (1) nach einem der vorhergehenden Ansprüche, wobei es sich bei der Kommunikationseinheit um eine Bluetooth-, Near-Field-Communication (NFC)- oder WLAN-Einheit handelt.
